# EUROPEAN PATENT SPECIFICATION

(11) **EP 2 671 553 B1**
(45) Date of publication and mention of the grant of the patent: **20.07.2016**
(21) Application number: 12741482.9
(22) Date of filing: 13.01.2012
(51) Int. Cl.: A61F 13/496, A61F 13/15, A61F 13/49, A61F 13/494

(54) **DISPOSABLE UNDERWEAR-TYPE ARTICLE FOR WEARING**
EINWEG-UNTERWÄSCHEARTIKEL
ARTICLE À PORTER DE TYPE SOUS-VÊTEMENT JETABLE

(30) Priority: 31.01.2011 JP 2011019076
(43) Date of publication of application: 11.12.2013
(73) Proprietor: Unicharm Corporation, Ehime 799-0111 (JP)
(72) Inventor: ICHIKAWA, Makoto, Kanonji-shi Kagawa 769-1602 (JP); SASAYAMA, Kenichi, Kanonji-shi Kagawa 769-1602 (JP); KATSURAGAWA, Kunihiko, Kanonji-shi Kagawa 769-1602 (JP); UKEGAWA, Kazuo, Kanonji-shi Kagawa 769-1602 (JP); NINOMIYA, Akihide, Kanonji-shi Kagawa 769-1602 (JP)
(74) Representative: Peter, Julian
(86) International application number: PCT/JP2012/050626
(87) International publication number: WO 2012/105293

(56) References cited:
- JP-A- 2004 329 590
- JP-B2- 4 240 463
- JP-U- H0 432 718
- US-A1- 2008 058 752
- US-B1- 6 217 563

## Description

### {Technical Field}

The present invention relates to disposable pull-on wearing articles used for various purposes and, more particularly, to disposable pull-on wearing articles such as diapers, toilet-training pants and incontinent briefs.

Conventionally, disposable pull-on wearing articles are known which include a waist panel adapted to cover the wearer's front and rear waist regions and a crotch panel connected to the waist panel, having an absorbent core assembly and adapted to cover the wearer's crotch.

For example, JP 4240463 B (PTL 1) discloses a disposable pull-on diaper in which the waist panel includes the front panel and the rear panel. In this diaper, the inner sheet lying on the skin-contactable side cooperates with the outer sheet lying on the non-skin-contactable side to form the front and rear panels and longitudinally extending opposite end edges of the crotch panel are respectively interposed between these inner and outer sheets.

The crotch panel includes the midsection in which the absorbent core assembly is placed, the side flaps formed by folding the crotch panel along the fold lines longitudinally extending along the opposite side edges of the midsection and the laminate sheet composed of plural sheets.

### {Citation List}

### {Patent Literature}

{PTL1}JP 4240463 B

Further prior art in this technical field is disclosed in patent US 6,217,563 B1.

### {Summary of Invention}

### {Technical Problem}

The crotch panel disclosed in PTL 1 is provided along the opposite side edges of the midsection with leg elastics extending in the longitudinal direction. These leg elastics are attached under tension to the opposite side edges of the midsection to be contractile.

In the diaper disclosed in PTL 1, however, the longitudinally opposite end edges of the leg elastics do not extend into the overlapping regions of the crotch panel and the waist panel. As a result, in these overlapping regions, the opposite side edges of the crotch panel may not be sufficiently kept in tight contact with the wearer's body and body exudates might leak out beyond the opposite side edges of the crotch panel. In other words, the invention disclosed in PTL 1 lacks important consideration of the possibility that body exudates might leak out beyond the opposite side edges of the crotch panel in the overlapping regions of the crotch panel and the waist panel.

Assuming that the opposite end edges of the leg elastics are arranged to extend to the overlapping regions of the crotch panel and the waist panel, the crotch panel should be attached under tension to the waist panel so that the crotch panel may contract in the longitudinal direction. In consequence, the crotch panel will be formed with gathers under its contractile force and these gathers will make it difficult to attach the crotch panel to the waist panel reliably. In addition, the presence of the elastics will make it difficult to keep the crotch panel in a flat state and make it further difficult to attach the crotch panel to the waist panel in a reliable manner.

Particularly in the disposable diaper having the crotch panel is attached to the outer side of the annular waist panel in order to improve the body exudates containment capacity, the diaper put on the wearer's body is subjected to the force acting to detach the crotch panel from the waist panel and therefore the crotch panel needs to be securely attached to the waist panel.

An obj ect of the present invention is to provide a disposable pull-on wearing article adapted to help prevent body exudates from leaking out beyond opposite side edges of a crotch panel and to allow a waist panel to be reliably attached to the waist panel.

### {Solution to Problem}

According to the present invention, there is provided a disposable pull-on wearing article including an annular waist panel having a skin-facing side and non-skin-facing side and a crotch panel connected to the waist panel wherein: the crotch panel includes an inner sheet lying on the skin-facing side, an outer sheet lying on the non-skin-facing side, a pair of side flaps and a pair of end flaps respectively extending along opposite side edges and opposite end edges of the inner and outer sheets, and paired sets of first elastics extending along the opposite side edges from one of the opposite end edges to a remaining end edge thereof substantially in a longitudinal direction wherein crossover regions of longitudinally opposite end portions of the side flaps and transversely opposite lateral portions are folded onto the skin-facing side; and the crossover regions define nonexistent regions in which none of the first elastics exists and the nonexistent regions are attached to the waist panel.

According to the present invention, the crotch panel includes fold lines extending in the longitudinal direction along the opposite side edges to define a midsection extending between the side flaps as viewed in the transverse direction and the side flaps, and the respective side flaps are folded onto the skin-facing side; and the paired sets of first elastics continuously extend from vicinities of the one end edge to vicinities of the remaining end edge and include portions intersecting with the respective fold lines of the opposite side edges of the midsection.

According to an embodiment of the present invention, in the respective side flaps, the paired sets of first elastics come closest to one another on a transverse imaginary center line bisecting the crotch panel in the longitudinal direction and gradually draw apart one from another as these first elastics draw apart from the transverse imaginary center line in the longitudinal direction.

According to still another embodiment of the present invention, the longitudinally opposite end edges of the first elastics are provided with non-stretched regions in which the first elastics are attached to the inner and outer sheets under no tension.

According to yet another embodiment of the present invention, the respective side flaps are provided with paired sets of second elastics to be spaced apart inwardly from the respective sets of first elastics with respect to the respective fold lines.

According to further another embodiment of the present invention, the skin-facing side of the midsection exposed between the respective side flaps is attached to the waist panel.

According to an additional embodiment of the present invention, the crotch panel is attached to the non-skin-facing side of the annular waist panel.

### {Advantageous Effects of Invention}

In the disposable pull-on wearing article according to the present invention, the side flaps are formed on the longitudinally opposite end edges with the nonexistent zones in which none of the elastics exists. As a consequence, the nonexistent zones develop none of gathers and are kept flat so that the crotch panel may be reliably attached to the waist panel. The crotch panel is additionally provided with the another pair of sets of elastics extending from the one end edge of the opposite end edges of the inner and outer sheets to the another end edge of the opposite end edges substantially in the longitudinal direction so that the opposite side edges of the crotch panel may contract even in the overlapping regions of the crotch panel and the waist panel. Consequently, it is also ensured to help prevent body exudates from leaking out beyond the opposite side edges of the crotch panel in these overlapping regions.

### {Brief Description of Drawings}

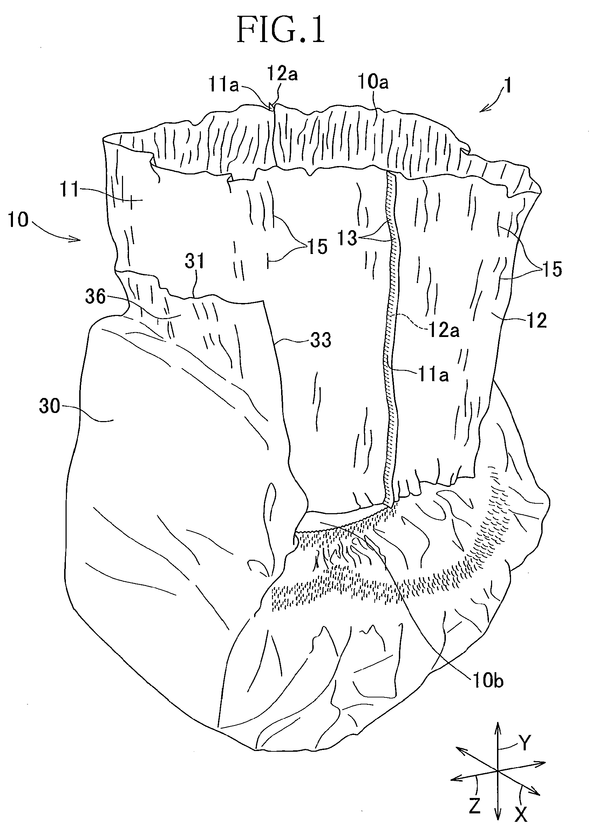

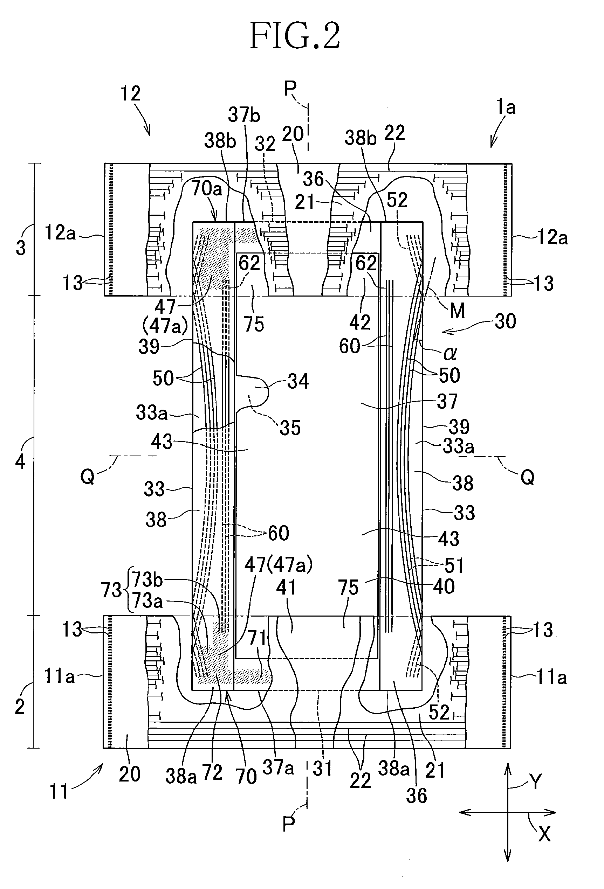

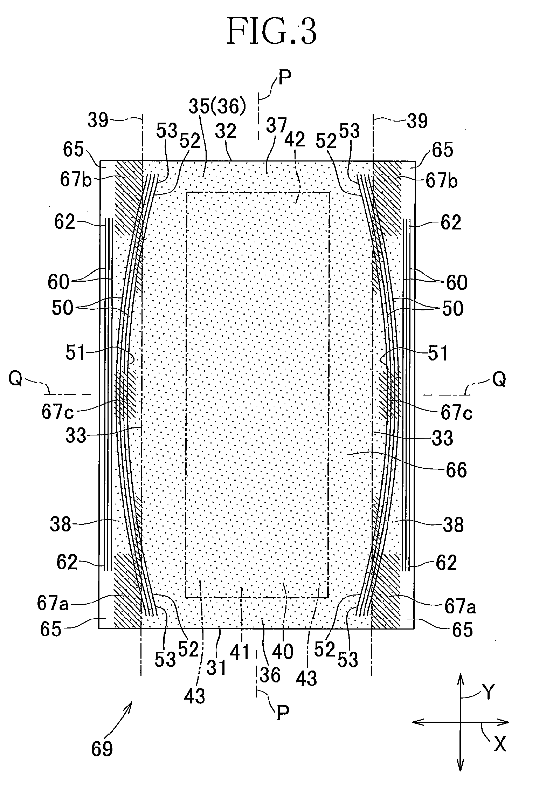

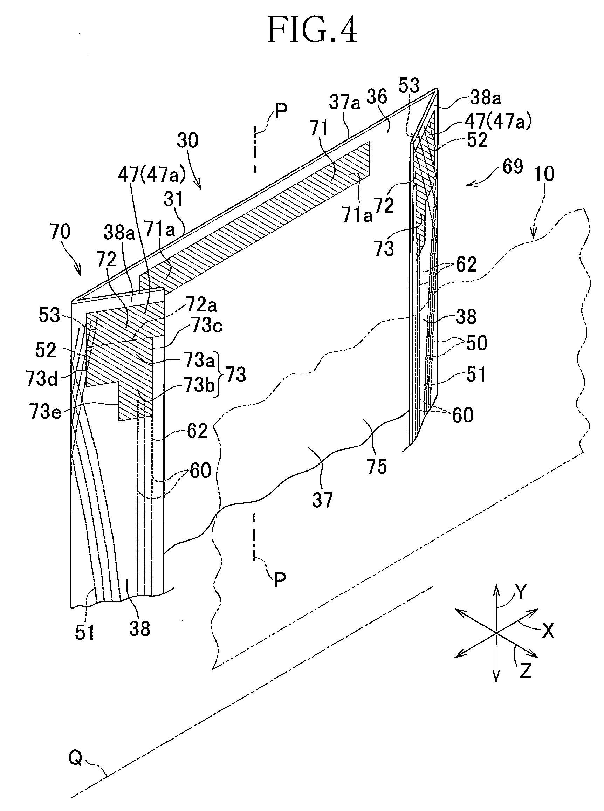

{Fig. 1} Fig. 1 is a perspective view of a diaper as one of typical examples of a disposable pull-on wearing article according to the present invention.
{Fig. 2} Fig. 2 is a partially cutaway developed plan view illustrating the diaper flatly developed and viewed from a skin-facing side.
{Fig. 3} Fig. 3 is a plan view of a laminate sheet.
{Fig. 4} Fig. 4 is a perspective view illustrating a manner in which a crotch panel is attached to a front panel.

### {Description of Embodiments}

An embodiment of a diaper as one of typical examples of a disposable pull-on wearing article according to the present invention will be described hereunder with reference to the accompanying drawings.

Fig. 1 is a partially cutaway perspective view illustrating a diaper 1 having a waist-opening 10a and a pair of leg-openings 10b as viewed from its front side.

In Fig. 1, X indicates a transverse direction, Y indicates a longitudinal direction being orthogonal to the transverse direction X and Z indicates a front-back direction being orthogonal to the transverse direction X and the longitudinal direction Y, respectively.

The diaper 1 includes an annular waist panel 10 extending in a waist circumferential direction and a crotch panel 30 extending in a direction orthogonal to the direction of the annular waist panel 10. The waist panel 10 includes a front panel 11 and a rear panel 12 respectively located on front and rear portions as viewed in the front-back direction Z wherein respective both side edges 11a, 12a of these front and rear panels 11, 12 are flatly joined together at seams 13 on both sides as viewed in the transverse direction X.

Fig. 2 is a partially cutaway plan view illustrating a developed diaper 1a obtained by, after the seams 13 have been opened, developing the diaper 1 in the transverse direction X as well as in the front-back direction Z wherein the developed diaper 1a is illustrated as viewed from a side facing the wearer' s skin (referred to hereinafter as a skin-facing side, and a side opposite to this skin-facing side will be referred to hereinafter as a non-skin-facing side).

In Fig. 2, P indicates a longitudinal imaginary center line bisecting the developed diaper 1a in the transverse direction X and Q indicates a transverse imaginary center line bisecting the crotch panel 30 in the longitudinal direction Y.

The diaper 1 includes a front waist region 2, a rear waist region 3 and a crotch region 4 being contiguous in the longitudinal direction Y. The front waist region 2 is adapted to cover the wearer's front waist, the rear waist region 3 is adapted to cover the wearer's rear waist and the crotch region 4 is adapted to cover the wearer's crotch.

The front and rear waist regions 2, 3 are defined by the front and rear panels 11, 12, respectively, and the crotch region 4 is defined by the crotch panel 30.

As illustrated, the front panel 11, the rear panel 12 and the crotch panel 30 are respectively symmetric about the longitudinal imaginary center line P-P.

The front and rear panels 11, 12 respectively include waist region's inner sheets 20 lying on the skin-facing side, waist region's outer sheets 21 lying on the non-skin-facing side and waist elastics interposed between these inner and outer sheets 20, 21, respectively. The waist region's inner and outer sheets 20, 21 are air-permeable and joined to each other, respectively, with not illustrated hot melt adhesives. The waist elastics 22 are secured under tension to at least one of the waist region's inner and outer sheets 20, 21 with a hot melt adhesive (not shown) in the front and rear panels 11, 12, respectively. In the state illustrated in Fig. 1, these waist elastics 22 contract in the transverse direction X and the waist region's inner and outer sheets 20, 21 are formed with wrinkles/gathers 15 under the effect of contractile force of the waist elastics 22.

The crotch panel 30 includes, as illustrated in Fig. 2, a crotch inner sheet (inner sheet) 34 lying on the skin-facing side and a crotch outer sheet (outer sheet) 35 lying on the non-skin-facing side. As material of the crotch inner sheet 34, a liquid-resistant and breathable plastic film may be used and as material of the crotch outer sheet 35, a liquid-impervious nonwoven fabric may be used. The crotch inner sheet 34 and the crotch outer sheet 35 are the same in the shape as well as in length dimensions measured in the transverse direction X and the front-back direction Z, respectively. The crotch inner and outer sheets 34, 35 are respectively contoured by crotch opposite end edges (opposite end edges) 31, 32 extending in the transverse direction X and crotch opposite side edges (opposite side edges) 33 extending in the longitudinal direction Y. The crotch panel 30 further includes a pair of side flaps 38 extending along the crotch opposite side edges 33 and front and rear end flaps 36 extending along the crotch opposite end edges 31, 32.

The crotch inner and outer sheets 34, 35 respectively have fold lines 39 extending in the longitudinal direction Y along the crotch opposite side edges 33 and these fold lines 39 divide the crotch inner and outer sheets 34 , 35 in the transverse direction X into a rectangular midsection 37 and the pair of side flaps 38 extending in the longitudinal direction Y wherein these side flaps 38 are folded onto the skin-facing side.

An absorbent structure 40 is affixed to the skin-facing side of the crotch inner sheet 34 by not illustrated bonding means such as hot melt adhesives. The absorbent structure 40 includes a liquid-absorbent core material and a liquid-pervious wrapping sheet used to wrap the core material. As the core material, fluff wood pulp, superabsorbent polymer particles or the like may be used. The absorbent structure 40 is contoured by liquid-absorbent opposite end portions 41, 42 extending in the transverse direction X and liquid-absorbent opposite side edge portions 43 extending in the longitudinal direction Y. The absorbent structure 40 is put in position so that the liquid-absorbent opposite end portions 41, 42 may lie inboard of the crotch opposite end edges 31, 32 in the longitudinal direction Y and the liquid-absorbent opposite side edge portions 43 may lie inboard of the respective fold lines 39 in the transverse direction X.

In Fig. 2, the crotch outer sheet 35 is eliminated only along the right side flap 38 to illustrate leg elastics (first and second elastics) 50, 60 so as to be exposed and the crotch outer sheet 35 is not eliminated along the left side flap 38 to illustrate the leg elastics 50, 60 so as to be not exposed. This is for convenience of illustration and obviously a pair of first leg elastics (first elastics) 50 and a pair of second leg elastics (second elastics) 60 are interposed between the crotch inner and outer sheets 34, 35 about the longitudinal imaginary center line P-P, respectively.

As materials of these leg elastics 50, 60, thread, strand or string elastic materials may be used. The leg elastics 50, 60 are secured to at least one of the crotch inner and outer sheets 34, 35 with not illustrated hot melt adhesives. In this embodiment of the diaper 1, each of the side edges 33 of the crotch panel 30 is provided with four of the first leg elastics 50 and three of the second leg elastics 60.

By providing the crotch panel 30 with a plurality of leg elastics 50, 60 in this manner, it is possible to increase regions in which the crotch panel 30 is put in close contact with the wearer's skin, thereby providing a good fit.

On the respective side flaps 38 of the developed diaper 1a, the first leg elastics 50 curve convexly toward the longitudinal imaginary center line P-P, more specifically, these first leg elastics 50 are closest to one another on the transverse imaginary center line Q-Q and gradually spaced one from another with distance from the transverse imaginary center line Q-Q in the longitudinal direction Y.

During use of the diaper 1, the transverse imaginary center line Q-Q preferably corresponds to the lowermost part of the crotch panel 30. Such arrangement ensures that the first leg elastics 50 are closest to one another at the lowest part of the crotch panel 30 as viewed in the longitudinal direction Y (See Fig. 1) and gradually spaced apart one from another with distance upwardly from the lowest part. As a consequence, in the vicinity of the wearer's groins, the first leg elastics 50 are closest to one another and gradually spaced apart one from another with distance outwardly from the groin region. In this way, a sufficiently good fit is ensured along the inner sides of the respective legs to help prevent body exudates such as urine from leaking, and it is also ensured that, along the outer sides of the respective legs, the wearer is free from an uncomfortable oppressive feeling.

The first leg elastics 50 preferably extend obliquely at a crossing angle α in a range of about 10 to about 90 degrees, more preferably about 15 to about 45 degrees defined between a tangent line M extending in the longitudinal direction and the respective crotch side edges 33.

As indicated by broken lines inFig. 2, the first leg elastics 50 extend to the vicinities of the opposite end edges 31, 32 of the midsection 37 so that these elastic 50 may cross the fold lines 39 of the respective side flaps 38.

The respective sets of the first leg elastics 50 are arranged along the opposite lateral edge portions of the midsection 37 to be most spaced one from another as they come near to the transverse imaginary center line Q-Q and to come gradually close to one another with distance from the transverse imaginary center line Q-Q.

The first leg elastics 50 continuously extend from a starting point in a vicinity of one of the crotch opposite end edges 31, 32, for example, from the end edge 31 upwardly in the midsection 37 as viewed in Fig. 2, then extendacross the respective fold lines 39 into the respective side flaps 38 in which these first leg elastics 50 extend further upwardly across the respective fold lines 39 again into the midsection 37 in which these first leg elastics 50 further extend upward as viewed in Fig. 2 to respective end edge points in the vicinity of the remaining end edge 32 of the crotch opposite end edges 31, 32. In this manner, these first leg elastics 50 continuously extend substantially along the crotch opposite side edges 33 in the longitudinal direction Y. In other words, the first leg elastics 50 continuously extend from the starting points in the vicinity of one of the crotch opposite end edges 31, 32 to the ending points in vicinities of the other of the crotch opposite end edges 31, 32.

The respective second leg elastics 60 are arranged in the developed diaper 1a so as to extend rectilinearly in the longitudinal direction Y. The paired sets of the second leg elastics are arranged to extend in parallel to each other. The respective sets of the second leg elastics 60 are arranged in the respective side flaps 38 to be interposed between the longitudinal imaginary center line P-P and the associated first leg elastics 50. In other words, a distance between the respective fold lines 39 and the associated second leg elastics 60 is larger than that between the respective fold lines 39 and the associated first leg elastics 50.

Fig. 3 illustrates a laminate sheet 69 composed of the crotch inner and outer sheets 34, 35 (see Fig. 2) between which the leg elastics 50, 60 are interposed and attached before the crotch inner and outer sheets 34, 35 are formed with the fold lines 39 wherein the crotch inner sheet 34 has been eliminated from the laminate sheet 69 for convenience of illustration. In this state, the respective second leg elastics 60 are positioned outboard of the associated first leg elastics 50, with these elastics 50, 60 being spaced apart farthest outwardly from the longitudianl imaginary center line P-P.

The paired sets of the first leg elastics 50 are arranged so as to be spaced apart from each other to the maximum extent on the transverse imaginary center line Q-Q, then to come gradually close to each other as these paired sets of the leg elastics 50 leave the transverse imaginary center line Q-Q in the longitudinal direction Y and to extend toward the longitudinal imaginary center line P-P in the vicinities of the crotch opposite end edges 31, 32. The first leg elastics 50 curve across the respective fold lines 39 so as to define middle portions 51 extending outboard of the respective fold lines 39 and end portions 52 extending inboard of the respective fold lines 39.

The first leg elastics 50 are provided in the vicinities of the crotch opposite end edges 31, 32 with non-stretched (relaxed) zones 53, respectively, in which the leg elastics 50 are attached under no tension to the crotch inner and outer sheets 34, 35 so that these zones 53 might exert no contractile force on the crotch inner and outer sheets 34, 35 and, except in the vicinity of the crotch opposite end edges 31, 32, the first leg elastics 50 are attached under tension to the crotch inner and outer sheets 34, 35 so that these first leg elastics 50 may exert contractile force on the crotch inner and outer sheet 34, 35.

The portions of the first leg elastics 50 adapted to exert the contractile force on the crotch inner and outer sheets 34, 35 are contractibly attached to these sheets 34, 35 under tension in a state being stretched to be twice the length of their initial (relaxed) length.

The non-stretched zones 53 preferably have a length dimension of 20 mm or longer and more preferably of 50 mm or longer in the longitudinal direction Y. The non-stretched zones 53 are provided preferably only in the midsection 37.

The second leg elastics 60 are provided in the end portions thereof with non-stretched (relaxed) zones 62, respectively, in which the elastics 60 are attached under no tension to the crotch inner and outer sheets 34, 35 so that these zones 62 may exert no contractile force on the crotch inner and outer sheets 34, 35 and, except in these end portions, the second leg elastics 60 are contractibly attached under tension to the crotch inner and outer sheets 34, 35 so that these elastics 60 may exert contractile force on the crotch inner and outer sheet 34, 35.

There are provided between the crotch inner and outer sheets 34, 35 with non-coated regions 65 in which any amount of hot melt adhesive is not applied, a low density coated region 66 in which an application amount of hot melt adhesives is reduced and high density coated regions 67 in which an application amount of hot melt adhesives is increased.

The non-coated regions 65 include the zones in which the end portions of the second leg elastics 60 extend but not include the zones in which the first leg elastics 50 are arranged and rectilinearly extend along the crotch opposite side edges 33 in the front-back region Z in Fig. 1, and in the longitudinal direction Y in Figs. 2, 3.

The low density coated region 66 extends over the entire areas of the crotch inner and outer sheet 34, 35 except the non-coated regions 65 and the high density coated regions 67 to be described below in more detail.

The high density coated regions 67 are arranged along the crotch opposite side edges 33 at intervals in the longitudinal direction Y between the respective fold lines 39 and the associated non-coated regions 65 and include a pair of front end edge coated regions 67a on the one end edge 31, a pair of rear end edge coated regions 67b on the other end edge 32 and a pair of middle coated regions 67c provided between the respective front end coated regions 67a and the respective rear end coated regions 67b as viewed in the longitudinal direction Y.

The leg elastics 50, 60 coated with hot melt adhesives in these coated regions 66, 67a, 67b, 67c are fixedly interposed in the predetermined shapes between the crotch inner sheet 34 and the crotch outer sheet 35 to form the laminate sheet 69 and thereafter the absorbent structure 40 is affixed to the skin-facing side of the crotch inner sheet 34 and the respective laminate sheets 69 are folded along the respective fold lines 39 to form the side flaps of the crotch panel 30.

Fig. 4 is a perspective view illustrating how to attach the crotch panel 30 and the waist panel 10 to each other.

The crotch panel 30 and the front panel 11 are attached to each other with hot melt adhesives applied to at least one of the crotch panel 30 and the front panel 11. While hot melt adhesives are applied only to the crotch panel 30 according to this embodiment, the adhesives may be applied only to the front panel 11 or to both the front panel 11 and the laminate sheet 69.

A coated region 70 in which the crotch panel 30 is coated with hot melt adhesives includes a first region 71 extending in the midsection 37 in the transverse direction X, a pair of second regions 72 rectilinearly extending to be spaced apart from opposite side edges 71a of the first region 71 as viewed in the transverse direction X so as to be drawn apart from the longitudinal imaginary center line P-P and a pair of third regions 73 arranged in the respective side flaps 38 to extend from respective lower end edges 72a of the second regions 72 as viewed in the longitudinal direction Y toward the transverse imaginary center line Q-Q.

Each of the third regions 73 includes a wide region 73a close to the one end edge 31 and a narrow region 73b spaced apart from the one end edge 31. On the side close to the longitudinal imaginary center line P-P, the wide region 73a and the narrow region 73b have a rectangular inner side edge 73c common to the both regions 73a, 73b. By contrast, on the side remote from the longitudinal imaginary center line P-P, an outer side edge 73e of the narrow region 73b is located on the inside of an outer side edge 73d of the wide region 73a. Inconsequence, the respective third regions 73 narrow as they come closer to the transverse imaginary center line Q-Q.

The first region 71 and the second regions 72 extending in the transverse direction X and the third regions 73 extending from the respective end edges of the second regions 72 toward the transverse imaginary center line Q-Q define the coated region 70 to form a pocket 75 (see Fig. 2) extending in the longitudinal direction Y between the crotch panel 30 and the waist panel 10. With such an arrangement, it is possible to collect body exudates discharged by the wearer in the recumbent position and thereby to improve a body exudates containment capacity. In this diaper 1, the skin-facing side of the crotch panel 30 is attached to the non-skin-facing side of the waist panel 10, and whereby the body exudates containment capacity may be further improved.

The middle portions 51 of the first leg elastics 50 is formed in a curved state to be spaced apart from the longitudinal imaginary center line P-P and to lie outboard of the respective third regions 73. In other words, the crossover regions 47 of the longitudinally opposite end portions of the respective side flaps 38 and the transversely opposite end portions of the respective end flaps 36 are formed with the nonexistent zones 47a in which none of the first leg elastics 50 is present and therefore the nonexistent zones 47a are free from development of gathers and remain flat. Also, none of the second leg elastics 60 is provided in the nonexistent zone 47a. In this way, the leg elastics 50, 60 should not interfere with attachment of the side flaps 38 to the front panel 11 and the crotch panel 30 may be reliably attached to the front panel 11.

In addition, the crotch panel 30 is provided with the first leg elastics 50 extending in the longitudinal direction Y from the vicinities of one of the opposite end edges 31, 32 of the crotch inner and outer sheets 34, 35 to the other of the opposite end edges 31, 32 so that, even in overlapping regions of the crotch panel 30 and the waist panel 10, the crotch opposite side edges 33 are contractile. Consequentially, it is possible to prevent body exudates from leaking out beyond the crotch opposite side edges 33 in these overlapping regions.

Furthermore, the opposite end portions 52 of the first leg elastics 50 extend into the midsection 37 which overlaps the nonexistent zones 47a of the respective side flaps 38 in the thickness direction of the crotch inner and outer sheets 34, 35. Such a unique arrangement allows contractile force of the first leg elastics 50 to be indirectly transferred to the front panel 11, and whereby ensures that body exudates should not leak out beyond the crotch opposite side edges 33.

In addition, the first region 71 of the midsection 37 exposed between the side flaps 38 is attached to the front panel 11 so that the front panel 11 may be further reliably attached to the crotch panel 30.

The crotch inner and outer sheets 34, 35 are the same in shape as well as in length dimensions in the longitudinal direction Y and the transverse direction X. As a consequence, folding of these sheets 34, 35 should not result in irregularities in the thickness direction. The side flaps flatly folded in this manner also ensure that the front panel 11 may be reliably attached to the crotch panel 30.

The end portions of the second leg elastics 60 lie in the narrow ranges 73b of the respective third regions 73 so that the contractile force of the second leg elastics 60 may be directly transferred to the waist panel 10. In consequence, during use of the diaper 1, the side flaps 38 are spaced apart upwardly from the midsection 37 under the contractile force of the second leg elastics 60 so that the side edges of the respective side flaps 38 may be put in close contact with the wearer's groins to help prevent leakage of body exudates.

In addition, as illustrated in Fig. 2, the leg elastics 50, 60 lie outboard of the absorbent structure 40 about the longitudinal imaginary center line P-P as viewed in the transverse direction X. With such an arrangement, the crotch opposite side edges 33 elasticized by the leg elastics 50, 60 are put in close contact about the wearer's legs to help prevent leakage of body exudates.

In addition to this, the crotch opposite side edges 33 are doubly-elasticized by the first leg elastics 50 and the second leg elastics 60 to help prevent leakage of body exudates further reliably.

Furthermore, the absorbent structure 40 is located inboard of the fold lines 39 as viewed in the transverse direction X about the longitudinal imaginary center line P-P. With such a placement of the absorbent structure 40, the absorbent structure 40 should not become bulky in the crotch region 4.

According to the illustrated embodiment, coated regions 70a in which the front panel 11 is coated with hot melt adhesives are different from the corresponding coated regions 70 in the crotch region 4 only in that the coated regions 70a include wide regions 73a but not narrow regions 73b. However, it is possible to configure the coated regions 70a in coincidence with the coated regions 70.

As one embodiment, the first leg elastics 50 have been described above to extend continuously from vicinities of the one end edge 31 to vicinities of the opposite end edge 32. However, as long as the first leg elastics 50 are configured to extend to the overlapping regions of the waist panel 10 and the crotch panel 30 to achieve the advantageous effect of the present invention which is to help prevent body exudates from leaking out beyond the opposite side edges 33 of the crotch panel 30 in the overlapping regions of the waist panel 10 and the crotch panel 30, it is not essential for the first leg elastics 50 to fully extend from the one end edge 31 to the other end edge 32 as has previously been described as long as these first leg elastics 50 extend to the overlapping regions of the waist panel 10 and the crotch panel 30. Though not illustrated, the first leg elastics 50 may be configured to fully extend between these two end edges 31, 32,

It is also unnecessary for the first leg elastics 50 to be continuous as long as the crossover regions 47 are free from the presence of these first leg elastics 50. In other words, it is possible to place a plurality of sets of elastics continually along the wearer's legs to configure the first leg elastics 50.

The present invention is not limited to diapers and applicable also to toilet-training pants or the like.

The component members of the diaper 10 are not limited to those described in this description but the other various types of material widely used in the relevant technical field may be used without limitation. The terms "first", "second" and "third" used in the description and claims of the present invention are used merely to distinguish the similar elements, similar positions or the other similar means.

### {Reference Signs List}

- 1: diaper (disposable pull-on wearing article)
- 10: waist panel
- 30: crotch panel
- 31: end edge
- 32: end edge
- 34: crotch inner sheet (inner sheet)
- 35: crotch outer sheet (outer sheet)
- 36: end flaps
- 37: midsection
- 38: side flaps
- 39: fold lines
- 47: crossover regions
- 47a: nonexistent zones
- 50: first leg elastics (elastics)
- 51: middle portions
- 52: end portions
- 53: non-stretched zones
- 60: second leg elastics (elastics)
- X: transverse direction
- Y: longitudinal direction
- P-P: longitudinal imaginary center line
- Q-Q: transverse imaginary center line

## Claims

1. A disposable pull-on wearing article comprising an annular waist panel (10) having a skin-facing side and non-skin-facing side and a crotch panel (30) connected to the waist panel (10) wherein:
the crotch panel (30) comprises an inner sheet (34) lying on the skin-facing side, an outer sheet (35) lying non the non skin facing side, a pair of side flaps (38) and a pair of end flaps (36) respectively extending along opposite side edges and opposite end edges of the inner and outer sheets (34, 35) and paired sets of first elastics (50) extending along the opposite side edges from one of the opposite end edges to the other end edge thereof substantially in a longitudinal direction wherein crossover regions (47) of longitudinally opposite end portions of the side flaps (38) and transversely opposite.laterals are folded onto the skin-facing side; and
the crossover regions (47) define nonexistent regions (47a) in which none of the first elastics exists and the nonexistent regions (47a) are attached to the waist panel (10), **characterized in that**
the crotch panel (30) includes fold lines (39) extending in the longitudinal direction along the opposite side edges to define a midsection (37) extending between the side flaps (38) as viewed in the transverse direction, and the respective side flaps (38) are folded onto the skin-facing side; and
the paired sets of first elastics (50) continuously extend from vicinities of the one end edge to vicinities of a remaining end edge and include portions intersecting with the respective fold lines (39) of the opposite side edges of the midsection (37).

2. The wearing article defined by Claim 1, wherein in the respective side flaps, the paired sets of first elastics (50) come closest to one another on a transverse imaginary center line bisecting the crotch panel (30) in the longitudinal direction and gradually draw apart one from another as these first elastics draw apart from the transverse imaginary center line in the longitudinal direction.

3. The wearing article defined by any one of Claims 1 through 2, wherein the longitudinally opposite end edges of the first elastics are provided with non-stretched regions in which the first elastics (50) are attached to the inner and outer sheets (34, 35) under no tension.

4. The wearing article defined by any one of Claims 1 through 3, wherein the respective side flaps (38) are provided with paired sets of second elastics (60) to be spaced apart inwardly from the respective sets of first elastics with respect to the respective fold lines (39).

5. The wearing article defined by any one of Claims 1 through 4, wherein the skin-facing side of the midsection (37) exposed between the respective side flaps (38) is attached to the waist panel (10).

6. The wearing article defined by any one of Claims 1 through 5, wherein the crotch panel (30) is connected to the non-skin-facing side of the annular waist panel (10).

## Patentansprüche

1. Einweg-Kleidungsstück zum Hineinschlüpfen, umfassend einen ringförmigen Taillenbereich (10), das eine der Haut zugewandte und eine der Haut abgewandte Seite und einen Schrittbereich (30) aufweist, der mit dem Taillenbereich (10) verbunden ist, wobei:
der Schrittbereich (30) eine innere Lage (34), die auf der der Haut zugewandten Seite aufliegt, eine äußere Lage (35), die auf der der Haut abgewandten Seite aufliegt, ein Paar Seitenklappen (38) und ein Paar Endklappen (36), die jeweils entlang der gegenüberliegenden Seitenkanten und gegenüberliegenden Endkanten der inneren Lagen (34) und der äußeren Lagen (35) verlaufen, sowie gepaarte Sätze erster elastischer Elemente (50), die entlang der gegenüberliegenden Seitenkanten von einer der gegenüberliegenden Endkanten zu der jeweils anderen Endkante im Wesentlichen in Längsrichtung verlaufen, umfasst, wobei Schnittbereiche (47) der in Längsrichtung einander gegenüberliegenden Endabschnitte der Seitenklappen (38) und der in Querrichtung einander gegenüberliegenden Seitenteile auf die der Haut zugewandte Seite gefaltet sind; und
die Schnittbereiche (47) nicht vorhandene Bereiche (47a) definieren, in denen keines der ersten elastischen Elemente vorhanden ist, und die nicht vorhandenen Bereiche (47a) am Taillenbereich (10) angebracht sind, **dadurch gekennzeichnet, dass**
der Schrittbereich (30) Faltlinien (39) umfasst, die in Längsrichtung entlang der gegenüberliegenden Seitenkanten verlaufen, um einen Mittelabschnitt (37) zu definieren, der in Querrichtung betrachtet zwischen den Seitenklappen (38) verläuft, und die entsprechenden Seitenklappen (38) auf die der Haut zugewandte Seite gefaltet sind; und
die gepaarten Sätze erster elastischer Elemente (50) kontinuierlich von Umgebungen der einen Endkante zu Umgebungen einer verbleibenden Endkante verlaufen und Bereiche umfassen, die die jeweiligen Faltlinien (39) der gegenüberliegenden Seitenkanten des Mittelabschnitts (37) schneiden.

2. Kleidungsstück nach Anspruch 1, wobei die gepaarten Sätze erster elastischer Elemente (50) auf den jeweiligen Seitenklappen auf einer gedachten Quermittellinie am nächsten aneinander liegen, die den Schrittbereich (30) in Längsrichtung halbiert, und graduell voneinander weg verlaufen, während die ersten elastischen Elemente von der gedachten Quermittellinie in Längsrichtung weg verlaufen.

3. Kleidungsstück nach einem der Ansprüche 1 bis 2, wobei die in Längsrichtung gegenüberliegenden Endkanten der ersten elastischen Elemente mit nicht-dehnbaren Bereichen ausgestattet sind, in denen die ersten elastischen Elemente (50) ohne Spannung an den inneren Lagen (34) und den äußeren Lagen (35) angebracht sind.

4. Kleidungsstück nach einem der Ansprüche 1 bis 3, wobei die jeweiligen Seitenklappen (38) mit gepaarten Sätzen zweiter elastischer Elemente (60) ausgestattet sind, die in Bezug auf die jeweiligen Faltlinien (39) nach innen und von den jeweiligen Sätzen erster elastischer Elemente beabstandet sind.

5. Kleidungsstück nach einem der Ansprüche 1 bis 4, wobei die der Haut zugewandte Seite des Mittelabschnitts (37), die zwischen den jeweiligen Seitenklappen (38) freilegt, am Taillenbereich (10) angebracht ist.

6. Kleidungsstück nach einem der Ansprüche 1 bis 5, wobei der Schrittbereich (30) mit der von der Haut abgewandten Seite des ringförmigen Taillenbereichs (10) verbunden ist.

## Revendications

1. Articule vestimentaire enfilable et jetable comprenant un pan annulaire de taille (10) présentant un côté orienté vers la peau et un côté non orienté vers la peau et un pan d'entrejambe (30) attaché au pan de taille (10), dans lequel :
le pan d'entrejambe (30) comprend une feuille interne (34) reposant sur le côté orienté vers la peau, une feuille extérieure (35) reposant sur le côté non orienté vers la peau, une paire de rabats latéraux (38) et une paire de rabats d'extrémité (36) s'étendant respectivement le long de bords latéraux opposés et de bords d'extrémité opposés des feuilles intérieure et extérieure (34, 35) et des ensembles appariés de premiers élastiques (50) s'étendant le long des bords du côté opposé d'un des bords d'extrémité opposée à l'autre bord d'extrémité de celui-ci, sensiblement dans une direction longitudinale où les régions de croisement (47) de parties d'extrémité longitudinalement opposées des rabats latéraux (38) et les parties latérales de côté transversalement opposé sont pliées sur le côté orienté vers la peau ; et
les régions de croisement (47) définissent des régions non existantes (47a) dans lesquelles aucun des premiers élastiques n'existe et où les régions non existantes (47a) sont attachées au pan de taille (10), **caractérisé en ce que**
le pan d'entrejambe (30) contient des lignes de pli (39) s'étendant dans la direction longitudinale le long des bords latéraux opposés pour définir une section médiane (37) s'étendant entre les rabats latéraux (38) tels qu'on les visualise dans la direction transversale, et où les rabats latéraux respectifs (38) sont pliés sur le côté orienté vers la peau ; et
les ensembles appariés de premiers élastiques (50) s'étendent continûment des voisinages du bord d'extrémité unique aux voisinages d'un bord d'extrémité résiduel et comprennent des portions coupant les lignes de pli respectives (39) des bords latéraux opposés de la section médiane (37).

2. Article vestimentaire selon la revendication 1, dans lequel dans les rabats latéraux respectifs, les ensembles appariés de premiers élastiques (50) viennent au plus près les uns des autres sur une ligne centrale imaginaire transversale coupant le pan d'entrejambe (30) dans la direction longitudinale et se séparent progressivement l'un de l'autre tandis que ces premiers élastiques se séparent de la ligne centrale imaginaire transversale dans la direction longitudinale.

3. Article vestimentaire selon l'une quelconque des revendications 1 à 2, dans lequel les bords d'extrémité longitudinalement opposés des premiers élastiques sont dotés de régions non étirées dans lesquelles les premiers élastiques (50) sont attachés aux feuilles intérieure et extérieure (34, 35) sous aucune tension.

4. Article vestimentaire selon l'une quelconque des revendications 1 à 3, dans lequel les rabats latéraux respectifs (38) sont dotés d'ensembles appariés de deuxièmes élastiques (60) à espacer vers l'intérieur à partir des ensembles respectifs de premiers élastiques par rapport aux lignes de pli respectives (39).

5. Article vestimentaire selon l'une quelconque des revendications 1 à 4, dans lequel le côté orienté vers la peau de la section médiane (37) exposé entre les rabats latéraux respectifs (38) est attaché au pan de taille (10).

6. Article vestimentaire selon l'une quelconque des revendications 1 à 5, dans lequel le pan d'entrejambe (30) est attaché au côté non orienté vers la peau du pan annulaire de taille (10).
